# EUROPÄISCHE PATENTSCHRIFT

(11) **EP 1 248 606 B1**
(45) Veröffentlichungstag und Bekanntmachung des Hinweises auf die Patenterteilung: **14.06.2006**
(21) Anmeldenummer: 00991160.3
(22) Anmeldetag: 11.12.2000
(51) Int. Cl.: A61K 31/15, A61P 25/28

(54) **VERWENDUNG VON FUMARSÄUREDERIVATEN ZUR BEHANDLUNG MITOCHONDRIALER KRANKHEITEN**
USE OF FUMARIC ACID DERIVATIVES FOR TREATING MITOCHONDRIAL DISEASES
UTILISATION DE DERIVES D'ACIDE FUMARIQUE POUR TRAITER DES MALADIES MITOCHONDRIALES

(30) Priorität: 10.01.2000 DE 10000577
(43) Veröffentlichungstag der Anmeldung: 16.10.2002
(73) Patentinhaber: Fumapharm AG, 6006 Luzern (CH)
(72) Erfinder: JOSHI, Rajendra, Kumar, CH-8048 Zürich (CH); STREBEL, Hans-Peter, CH-6006 Luzern (CH)
(74) Vertreter: Schwabe - Sandmair - Marx
(86) Internationale Anmeldenummer: PCT/EP2000/012504
(87) Internationale Veröffentlichungsnummer: WO 2001/051047

(56) Entgegenhaltungen:
- WO-A-95/25102
- WO-A-99/21565
- CA-A- 2 248 955
- US-A- 4 746 668
- US-A- 5 538 968

## Beschreibung

Die Erfindung betrifft die Verwendung einzelner Fumarsäurederivate oder deren Mischungen zur Herstellung einer pharmazeutischen Zubereitung zur Behandlung mitochondrialer Erkrankungen, insbesondere zur Behandlung des Parkinson-Syndroms, der Alzheimer-Krankheit, der Chorea-Huntington-Krankheit, der Retinopathia pigmentosa und der mitochondrialen Enzephalomyopathie.

Die Mitochondrien verfügen über ein selbständiges genetisches System aus DNA (mtDNA) und RNA und können daher gewisse Proteine selbst synthetisieren. Sowohl Gene des Zellkerns als auch das mitochondriale Genom codieren für die Komponenten der oxidativen Phosphorylierung sowie des Citratzyklus'. Ein genetischer Defekt der mtDNA kann somit die oxidative Phosphorylierung bzw. den Citratzyklus beeinträchtigen und zu Fehlfunktionen führen. Derartige Defekte bzw. Fehlfunktionen sind mit den sog. mitochondrialen Erkrankungen in Verbindung gebracht worden.

Genetische Defekte der mtDNA können durch Punktmutationen entstehen, bei denen eine Base durch eine andere ersetzt wird. Diese Punktmutationen werden beispielsweise mit neurogenetischer Muskelschwäche, Ataxie und der Retinopathia pigmentosa in Verbindung gebracht.

Genetische Defekte der mtDNA können auch durch Insertions- oder Deletionsmutationen verursacht werden, bei denen ein oder mehrere Nucleotidpaare in die DNA eingeschoben oder aus ihr getilgt werden. Dieser Mutationsmechanismus wird im Zusammenhang mit dem Kearns-Sayre-Syndrom und dem Pearson-Syndrom diskutiert.

Die Mutation von mtDNA spielt auch bei neurodegenerativen Krankheiten wie dem Parkinson-Syndrom, der Alzheimer-Krankheit oder der Chorea-Huntington-Krankheit eine Rolle (Encyclopedia of Molecular Biology and Molecular Medicine, Vol. 4. Ed. R.A. Meyers). Allerdings konnten diesen Krankheiten die ursächlichen Mutationen bisher nicht zweifelsfrei zugeordnet werden. Diskutiert wird bspw. auch eine Akkumulation von Mutationen als Basis der Pathogenese.

Das Parkinson-Syndrom weist eine Vielzahl von Symptomen auf, die sich in drei Gruppen unterteilen lassen. Motorische Störungen äußern sich durch die Plus-Symptome Rigor (Tonusvermehrung der quergestreiften Muskulatur) und mittel- bis grobschlägigen Tremor (rasch aufeinanderfolgende Zuckungen) sowie das Minus-Symptom Hypo- bzw. Akinese (Verarmung der Gesamtmotorik, Verlust der Stellreflexe). Desweiteren beobachtet man vegetative Symptome (vermehrter Speichel- und Tränenfluß, Salbengesicht) und psychische Störungen (erschwerte Entschlussfähigkeit, depressive Verstimmung u.a.).

Das Leiden beruht auf einem Untergang von Nervenzellen in den motorischen Kerngebieten des Hirnstamms. In Deutschland sind ca. 200.000 Patienten davon betroffen. Auf molekularer Ebene wird das Parkinson-Syndrom u.a. mit Mutationen des mitochondrialen Genoms in Verbindung gebracht. Bei Parkinson-Patienten konnten mtDNA-Deletionen nachgewiesen werden. Weiterhin kommt es beim Parkinson-Syndrom zu einer Verarmung bestimmter Hirngebiete an Dopamin. Die beobachteten Symptome sind Ausdruck des gestörten Gleichgewichts zwischen den neurohumoralen Transmittersubstanzen Acetylcholin und Dopamin.

Ansatzpunkte einer medikamentösen Therapie sind derzeit die Hemmung der cholinergen Neurotransmission mit zentral wirksamen Anticholinergika, die Erhöhung der Dopamin-Konzentration durch Gabe der Dopamin-Vorstufe Levodopa oder auch die Stimulation zentraler Dopamin-Rezeptoren mit direkten dopaminergen Agonisten.

Konkrete Therapien umfassen daher die Gabe von Anticholinergika oder von Levodopa. Um bei Parkinsonkranken sowohl die Plus-Symptome als auch das Minus-Symptom günstig zu beeinflussen, ist meist eine Kombinationstherapie erforderlich, die zudem durch nichtmedikamentöse Therapiemaßnahmen ergänzt wird.

Andererseits ist bei Parkinsonkranken, die bereits unter deutlichen psycho-organischen Störungen oder exogen-psychotischen Symptomen leiden die Therapie mit Anticholinergika kontraindiziert, da mit einer Verstärkung der Symptomatik gerechnet werden muß.

Bei der Therapie mit Levodopa treten als den therapeutischen Einsatz beschränkende Nebenwirkungen motorische Symptome (Hyperkinesen, Dyskinesen), vegetative Störungen (u. a. Magen-Darm-Beschwerden) und kardiovaskuläre Störungen (z. B. orthostatische Beschwerden) auf.

Die Alzheimer-Krankheit ist eine irreversibel fortschreitende präsenile oder senile Demenz, die durch Zerstörung von Gehirnarealen gekennzeichnet ist. Neben den Einflüssen von mitochondrialen Gendefekten zeigen neuere Untersuchungen, dass ein vermehrtes Vorkommen des Apolipoproteins E₄ (apo E₄) mit dem Auftreten der Alzheimer-Krankheit in Zusammenhang steht. Bei der erblichen Form dieser Krankheit ist das entsprechende Gen für das apo E₄ häufig defekt. Anders als beim Parkinson-Syndrom stehen für die Alzheimer-Krankheit bisher mit Ausnahme der Indometacin-Behandlung keine Therapien zur Verfügung. Indometacin verursacht jedoch ebenfalls erhebliche Nebenwirkungen.

Die Retinopathia pigmentosa ist ein meist erblicher, selten erworbener degenerativer Prozess, der mit einer Engstellung der Netzhautgefäße, Optikusatrophie, dem Untergang der nervalen Elemente der Netzhaut und einer Ablagerung von Pigmenten verbunden ist. Als Symptome treten Nyktalopie, erhebliche Gesichtsfeldeinengung und Erblindung auf.

Bei der mitochondrialen Enzephalomyopathie handelt es sich um eine Erkrankung, bei der Störungen der mitochondrialen Atmungskette vorliegen. Typische Symptome sind Myopathie (sog. ragged red fibres myopathy), Minderwuchs, Demenz, epileptische Anfälle, Ataxie, neurologische Herdstörungen und MELAS (mitochondriale Enzephalomyopathie, Lactat-Acidose und Schlaganfälle).

Chorea-Huntington ist eine autosomal-dominant erbliche Erkrankung mit einem Defekt auf dem kurzen Arm des Chromosoms 4, die sich meist zwischen dem 30. und 50. Lebensjahr manifestiert und mit progressiver Demenz verbunden ist. Als Ursache ist eine Schädigung oder Atrophie des Nucleus candatus und eventuell des Nucleus lentiformis zu nennen. Eine Störung des Neurotransmitterstoffwechsels sowie der Einfluss von mtDNA-Defekten werden diskutiert.

Die WO 99/21565 offenbart Nahrungsergänzungsmittel für Patienten, die an zerebralen Stoffwechselinsuffizienzen leiden. Gemäß dieser Offenbarung wird eine pharmazeutische Zubereitung verabreicht, die einen Zucker und einen Krebszyklus-Intermediat oder ein Salz davon oder auch einen Vorläufer eines solches Intermediats enthält. Die Krebszyklus-Intermediate schließen Zitronensäure, Aconitinsäure, Isozitronensäure, α-Ketoglutarsäure, Bemsteinsäure, Fumarsäure, Äpfelsäure und Oxalessigsäure sowie Mischungen davon ein. Gelehrt wird weiterhin, dass die zerebralen Stoffwechselinsuffizienzen mit einem Zusammenbruch des Energiegewinnungsmetabolismus der Zelle diese Zellen assoziert sind, damit an einem Mangel an einer leicht verfügbaren Energieform (ATP) leiden. In den Beispielen veranschaulicht werden lediglich Äpfelsäure enthaltende Zubereitungen.

Eine Aufgabe der Erfindung besteht demnach darin, eine pharmazeutische Zubereitung zur Behandlung mitochondrialer Krankheiten, insbesondere der vorstehend genannten Krankheiten bereitzustellen und so eine teilweise, bisher nicht mögliche medikamentöse Behandlung dieser Krankheiten zu ermöglichen. Eine weitere Aufgabe der Erfindung besteht darin, eine pharmazeutische Zubereitung zur Behandlung der genannten Krankheiten bereitzustellen, die die medikamentösen Nebenwirkungen bisheriger Therapien reduziert und keine Kombinationstherapie erfordert.

Die Lösung der erfindungsgemäßen Aufgabe liegt in der Verwendung einzelner oder einer Mischung von Fumarsäurederivate(n) zur Herstellung einer pharmazeutischen Zubereitung, wie in Ansprüch 1 definiert, zur Behandlung mitochondrialer Krankheiten, insbesondere zur Behandlun der Alzhelmer-Krankheit, des Parkinson-Syndroms, der Chorea-Huncington-Kranlcheit, der Retinopathia pigmentosa oder der mitochondrialen Enzephalomyopathie. Die erfindungsgemäßen Gegenstände sind in den Ansprüchen im Einzelnen gekennzeichnet.

Bekannt ist, dass pharmazeutische Zubereitungen, die nach Verabreichung bei ihrem biologischen Abbau in den Zitronensäurezyklus einmünden oder diesem angehören, zumeist in hoher Dosierung immer mehr an therapeutischem Wert gewinnen, da man mit ihrer Hilfe kryptogenetisch bedingte Krankheiten zu lindern oder zu heilen vermag.

So hemmt Fumarsäure das Wachstum des Ehrlich-Ascites-Tumors bei Mäusen, vermindert die toxischen Effekte von Mitomycin C und Aflafoxin und besitzt eine antipsoriatische sowie antimikrobielle Wirkung. Allgemein ist die Behandlung von Psoriasis mit verschiedenen Fumarsäurederivaten bereits in einer Anzahl von Patenten beschrieben worden, siehe z. B. EP 188 749, DE 25 30 372, DE 26 21 214 oder EP 312 697.

Eine weitere Verwendung bestimmter Fumarsäurederivate, nämlich der Alkylhydrogenfumarate, offenbaren die DE 197 21 099.6 sowie die DE 198 53 487.6 gemäß denen diese bestimmten Fumarsäurederivate zur Behandlung von Autoimmunerkrankungen wie insbesondere der Polyarthritis, der Multiplen Sklerose und von Graft-versus-Host-Reaktionen beschrieben werden. Weiterhin lehren die DE 198 53 487.6 sowie die DE 198 39 566.3 die Verwendung von Alkylhydrogenfumaraten und Diallcylfumaraten in der Transplantationsmedizin.

Es wurde nun überraschend gefunden, dass einzelne Fumarsäurederivate oder deren Mischungen, wie in Ansprüch 1 definiert, vorteilhaft zur Herstellung einer pharmazeutischen Zubereitung zur Behandlung mitochondrialer Erkrankungen, insbesondere zur Behandlung des Parkinson-Syndroms, der Alzheimer-Krankheit, der Chorea-Huntington-Krankheit, der Retinopathia pigmentosa oder der mitochondrialen Enzephalomyopathie verwendet werden können.

Erfindungsgemäß verwendet man zur Herstellung der pharmazeutischen Zubereitung einen oder mehrere Fumarsäuredialkylester und/oder Fumarsäuremonoalkylester in Form der freien Säure oder in Salzform.

Die Fumarsäuredialkylester entsprechen der Formel 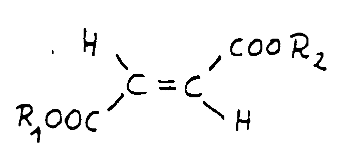 in der R₁ und R₂, die jeweils gleich oder verschieden sein können, unabhängig voneinander einen linearen, verzweigten, oder cyclischen, gesättigten oder ungesättigten C₁₋₂₄-Alkylrest oder einen C₅₋₅₀-Arylrest bedeuten und diese Reste gegebenenfalls mit Halogen (F, Cl, Br, I), Hydroxy, C₁₋₄-Alkoxy, Nitro oder Cyano substituiert sind.

Bevorzugt handelt es sich bei den Resten R₁ und R₂ um Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- und/oder 3-Hydroxypropyl, 2-Methoxyethyl, Methoxymethyl oder 2- oder 3-Methoxypropyl.

Die Fumarsäuremonoalkylester entsprechen der Formel 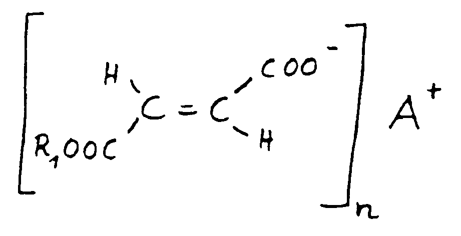 in der R₁ einen wie oben definierten Rest bedeutet; A Wasserstoff oder ein Alkali- oder Erdalkalimetallkation oder ein physiologisch verträgliches Übergangsmetallkation, vorzugsweise ausgewählt unter Li⁺, Na⁺, K⁺, Mg²⁺, Ca²⁺, 2n²⁺, Fe²⁺, Mn²⁺, ist und n gleich 1 oder 2 ist und der Valenz von A entspricht.

Die Fumarsäurederivate gemäß der Erfindung werden in einer solchen Menge eingesetzt, dass die pharmazeutische Zubereitung pro Dosiseinheit eine Menge von einem oder mehreren Fumarsäurederivat(en) enthält, die einer Menge von 1-500 mg, vorzugsweise 10-300 mg und am meisten bevorzugt 10-200 mg Fumarsäure entspricht bzw. äquivalent ist.

Bevorzugt sind Anwendungen, bei denen die pharmazeutische Zubereitung oral, parenceral, rektal, transdermal oder ophthal (in Form von Augentropfen) verabreicht wird, wobei die orale Verabreichung bevorzugt ist. Die Zubereitung liegt dann in für die jeweilige Verabreichung geeigneter Form vor.

Erfolgt eine orale Verabreichung, so liegt eine pharmazeutische Zubereitung gemäß der Erfindung bevorzugt in Form von Unit-Dose-Tabletten, gegebenenfalls verkapselten oder in Sackets abgefüllten Mikrotabletten (Mikropellets) bzw. Granulat, Kapseln oder Trinklösungen vor. Wenn es sich um feste Dosisformen bzw. Verabreichungsformen handelt, werden diese in einer bevorzugten Ausführungsform mit einem magensaftresistenten Überzug versehen. Der Überzug kann auch auf den verkapselten bzw. abgefüllten Dosisformen vorgesehen sein.

Bevorzugt verwendet werden gemäß der Erfindung ein oder mehrere Fumarsäurederivat(e), die aus der Gruppe, umfassend Fumarsäuredlmethylester, Fumarsäurediethylester, Fumarsäuremethylethylester, Methylhydrogenfumarat, Ethylhydrogenfumarat, Magnesiummethytfumarat, Magnesiumethylfumarat, Zinkmethylfumarat, Zinkethylfumarat, Eisenmethylfumarat, Eisenethylfumarat, Calciummethylfumarat und/oder Calciumethylfumarat ausgewählt sind.

Die erfindungsgemäße pharmazeutische Zubereitung kann bevorzugt 10-500 mg Dialkylfumarat, insbesondere Dimethylfumarat und/oder Diethylfumarat, 10-500 mg Calciumalkylfumarat, insbesondere Calciummethylfumarat und/oder Calciumethylfumarat, 0-250 mg Zinkalkylfumarat, insbesondere Zinkmethylfumarat und/oder Zinkethylfumarat, 0-250 mg Alkylhydrogenfumarat, insbesondere Methylhydrogenfumarat und/oder Ethylhydrogenfumarat und 0-250 mg Magnesiumalkylfumarat, insbesondere Magnesiummethylfumarat und/oder Magnesiumethylfumarat enthalten, wobei die Summe der genannten Mengen einem Äquivalent von 500 mg, vorzugsweise 300 mg und am meisten bevorzugt 200 mg Fumarsäure entspricht.

Bevorzugte Zubereitungen gemäß der Erfindung enthalten ausschließlich Methylhydrogenfumarat oder Dimethylfumarat in einer Menge von 10 bis 300 mg.

Die in den erfindungsgemäßen Zubereitungen enthaltenen Fumarsäurederivate werden beispielsweise gemäß dem in der EP 0 312 679 beschriebenen Verfahren hergestellt.

Im Folgenden werden zur Erläuterung der erfindungsgemäßen Verwendung verschiedene Beispiele für die Herstellung bevorzugter Arzneimittel gegeben:

### Beispiel 1

Herstellung von Filmtabletten mit magensaftresistentem Überzug enthaltend 100,0 mg Monomethylfumarat-Ca-Salz, entsprechend 78 mg Fumarsäure.

10,000 kg Monomethylfumarat-Ca-Salz werden zerkleinert, intensiv gemischt und unter entsprechenden Vorsichtsmaßnahmen (Atemmaske, Handschuhe, Schutzanzug etc.) mittels eines Siebes 800 homogenisiert. Anschließend wird ein Hilfsstoffgemisch folgender Zusammensetzung hergestellt: 21,000 kg Stärkederivat (STA-RX 1500® ), 2,000 kg mikrokristalline Cellulose (Avicel PH 101® ), 0,600 kg Polyvinylpyrrolidon (PVP, Kollidon® 25), 4.000 kg Primogel® , 0,300 kg kollodiale Kieselsäure (Aerosil® ).

Das gesamte Pulvergemisch wird mit dem Wirkstoff versetzt, gemischt, mittels eines Siebes 200 homogenisiert und mit einer 2 %-igen wäßrigen Lösung von Polyvinylpyrrolidon (PVP, Kollidon® 25) auf übliche Weise zu einem Bindemittelgranulat verarbeitet und in trockenem Zustand mit der äußeren Phase gemischt. Diese besteht aus 2,000 kg eines sogenannten FST-Komplexes, enthaltend 80 % Talk, 10 % Kieselsäure und 10 % Magnesiumstearat.

Es wird anschließend auf übliche Weise zu gewölbten Tabletten von 400 mg Gewicht und 10,0 mm Durchmesser gepreßt. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode.

### Magensaftresistenz

Es wird eine Lösung von 2,250 kg Hydroxypropylmethylcellulosephthalat (HPMCP. Pharmacoat HP® 50) in einem Lösungsmittelgemisch von 2,50 l demineralisiertem Wasser, 13,00 l Aceton Ph.Helv. VII und 13,00 l Ethanol (94 Gewichtsprozent) gelöst und die Lösung mit 0,240 kg Rizinusöl (Ph.Eur. II) versetzt. Die Lösung wird im Dragierkessel auf traditionelle Weise in Portionen auf die Tablettenkerne aufgeleert oder aufgesprüht bzw. in einem Wirbelschichtapparat entsprechender Konstruktion aufgetragen.

Nach entsprechender Trocknung wird anschließend der Filmüberzug angebracht. Dieser setzt sich zusammen aus einer Lösung von Eudragit E® 12,5 % 4,800 kg, Talcum Ph. Eur. II 0,340 kg, Titan (VI)-oxid Cronus RN 56® 0,520 kg, Farblack ZLT-2 blau (Siegle) 0,210 kg und Polyethylenglycol 6000 Ph. Helv. VII 0,120 kg in einem Lösungsmittelgemisch von 8,200 kg 2-Propanol Ph.Helv. VII. 0,060 kg Glycerintriacetat (Triacetin® und 0,200 kg Aqua demineralisata. Nach homogener Verteilung im Dragierkessel oder Wirbelschichtbett wird getrocknet und auf übliche Weise poliert.

### Beispiel 2

Herstellung von magensaftresistenten Kapseln, enthaltend 86,5 mg Monoethylfumarat-Ca-Salz und 110,0 mg Dimethylfumarat, entsprechend insgesamt 150 mg Fumarsäure. 8,650 kg Monoethylfumarat-Ca-Salz und 11,000 kg Dimethylfumarat werden mit einem Gemisch bestehend aus 15,000 kg Stärke, 6,000 kg Lactose Ph. Helv. VII, 2,000 kg mikrokristalliner Cellulose (Avicel® ), 1,000 kg Polyvinylpyrrolidon (Kollidon® 25) und 4,000 kg Primogel® intensiv gemischt und unter entsprechenden Vorsichtsmaßnahmen (Atemmaske, Handschuhe, Schutzanzug etc.) mittels eines Siebes 800 homogenisiert.

Das gesamte Pulvergemisch wird mit einer 2 %-igen wäßrigen Lösung von Polyvinylpyrrolidon (Kollidon® 25) auf übliche Weise zu einem Bindemittelgranulat verarbeitet und in getrocknetem Zustand mit der äußeren Phase gemischt. Diese besteht aus 0,350 kg kolloidaler Kieselsäure (Aerosil® ), 0,500 kg Magnesiumstearat und 1,500 kg Talkum Ph. Helv. VII. Das homogene Gemisch wird anschließend in entsprechende Kapseln in Portionen von 500,0 mg abgefüllt, welche abschließend auf übliche Weise mit einem magensaftresistenten Überzug, bestehend aus Hydroxypropylethylcellulosephatalat und Rizinusöl als Weichmacher, versehen werden. Die Abfüllung kann ebenfalls anstelle von Hartgelatinekapseln in entsprechende magensaftresistente Kapseln, bestehend aus einem Gemisch von Cellulloseacetatphthalat (CAP) und Hydroxypropylethylcellulosephthalat (HPMCP), erfolgen.

### Beispiel 3

Herstellung von magensaftresistenten Mikrotabletten in Kapseln, enthaltend 87,0 Monoethylfumarat Ca-Salz, 120 mg Dimethylfumarat, 5,0 mg Monoethylfumarat Mg-Salz und 3,0 mg Monoethylfumarat Zn-Salz, entsprechend insgesamt 164 mg Fumarsäure ("Forte"-Tabletten).

8,700 kg Monoethylfumarat Ca-Salz, 12,000 kg Dimethylfumarat, 0,500 kg Monoethylfumarat Mg-Salz, 0,30 kg Monoethylfumarat Zn-Salz werden zerkleinert, intensiv gemischt und mittels eines Siebs 800 unter entsprechenden Vorsichtsichtsmaßnahmen (Atemmaske, Handschuhe, Schutzanzug, etc.) homogenisiert. Es wird ein Hilfsstoffgemisch folgender Zusammensetzung hergestellt: 18,00 kg Stärkederivat (STA-RX 1500), 0,30 kg Cellulose mikrokristallin (Acivel PH 101), 0,75 kg PVP (Kollidon 120), 4,00 kg Primogel, 0,25 kg Kieselsäure kolloidal (Aerosil). Das gesamte Pulvergemisch wird mit dem Wirkstoffgemisch versetzt und mittels eines Siebes 200 homogenisiert und mit einer 2%-igen wäßrigen Lösung von Polyvinylpyrrolidon (Kollidon K25) auf übliche Weise zu einem Bindemittelgranulat verarbeitet und in trockenem Zustand mit der äußeren Phase gemischt. Diese besteht aus 0,50 kg Magnesiumstearat und 1,50 kg Talkum. Das Pulvergemisch wird anschließend auf übliche Weise zu gewölbten Mikrotabletten von 10,0 mg Bruttomasse und 2,0 mm Durchmesser gepreßt. Anstelle dieser klassischen Tablettiermethode können auch andere Methoden zur Herstellung von Tabletten verwendet werden, wie Dirzkttablettierung sowie feste Dispersionen nach der Schmelzmethode und die Sprühtrocknungsmethode.

Der magensaftresistente Überzug kann in einem klassischen Dragierkessel aufgeleert oder aufgesprüht sowie in einer Wirbelschichtapparatur aufgebracht werden. Zum Erreichen der Magensaftresistenz wird portionsweise eine Lösung von 2,250 kg Hydroxypropylmethylcellulosephthalat (HPMCP, Pharmacoat HP 50), in einem Gemisch folgender Lösungsmittel aufgelöst: Aceton 13,00 1, Ethanol 94 Gewichtsprozent denaturiert mit 2 % Keton 13,50 l und Aqua demineralisata 2,50 l. Zu der fertigen Lösung wird als Weichmacher Rizinusöl 0,240 kg zugegeben und auf übliche Weise in Portionen auf die Tablettenkerne aufgetragen.

Filmcoat: Nach beendeter Trocknung wird anschließend in der gleichen Apparatur eine Suspension folgender Zusammensetzung als Filmcoat aufgetragen: Talk 0,340 kg, Titan (VI)-oxid Cronus RN 56 0,400 kg, Farblack L-Rotlack 86837 0,324 kg, Eudragit E 12,5 % 4,800 kg und Polyethlenglycol 6000 pH 11 XI 0,120 kg in einem Lösungsmittelgemisch folgender Zusammensetzung: 2-Propanol 8,170 kg, Aqua demineralisata 0,200 kg und Glycerintracetat (Triacetin) 0,600 kg.

Die magensaftresistenten Mikrotabletten werden anschließend in Hartgelantine-Steckkapseln zu 500,0 mg netto Gewicht eingefüllt und verschlossen.

### Beispiel 4

Beispiel 4 zeigt den stimulierenden Einfluß von Fumarsäurederivaten auf die Enzymaktivität der Succinat-Dehydrogenase.

Die Succinat-Dehydrogenase ist Bestandteil der Mitochondrienmembran und katalysiert innerhalb des Citratzyklus die Dehydrierung von Bernstein zu Fumarsäure. Der Wasserstoff wird über das Elektronentransfer-Flavoprotein an die Atmungskette weitergegeben. Somit kann über die Aktivität der Succinat-Dehydrogenase der Elektronenfluß der E-lektronentransportkette beeinflußt werden. Letztere ist wiederum mit dem Vorgang der oxidativen Phosphorylierung verknüpft, dessen Störung als eine Ursache mitochondrialer Krankheiten angesehen wird. Eine Beeinflussung der Aktivität der Succinat-Dehydrogenase kann sich folglich auch auf die oxidative Phosphorylierung auswirken.

Die folgende Tabelle 1 zeigt eine Bewertung des stimulierenden Einflusses von Fumarsäurederivaten auf die Enzymaktivität der Succinat-Dehydrogenase.

| **Fumarate** | **Fibroblasten [0.75m Val/l]** |
|---|---|
| Dimethylfumarat | stark (838 %) |
| Calcium-Monoethylfumarat | mittel |
| Magnesium-Monoethylfumarat | schwach |
| Zink-Monoethylfumarat | stark (107 %) |
| Monoethylfumarat | schwach |

## Patentansprüche

1. Verwendung einzelner oder einer Mischung von Fumarsäurederivaten zur Herstellung einer pharmazeutischen Zubereitung zur Behandlung mitochondrialer Erkrankungen, worin es sich bei dem oder den Fumarsäurederivaten um solche, ausgewählt aus der Gruppe, bestehend aus Fumarsäuredialkylestem und Fumarsäurerüonoalkylestern in Form der freien Säure oder eines Salzes davon, handelt, wobei der Fumarsäuredialkylester der Formel 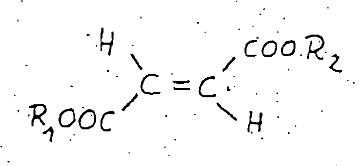 entspricht, worin R₁ und R₂, die jeweils gleich oder verschieden sein können, unabhängig voneinander einen linearen, verzweigten, cyclischen, gesättigten oder ungesättigten C₁₋₂₄-Alkylrest oder einen C₅₋₂₀ Arylrest bedeuten und diese Reste gegebenenfalls mit Halogen (F, Cl, Br, I), Hydroxy, C₁₋₄-Alkoxy, Nitro oder Cyano substituiert sind, oder wobei der Fumarsäuremonoalkylester der Formel 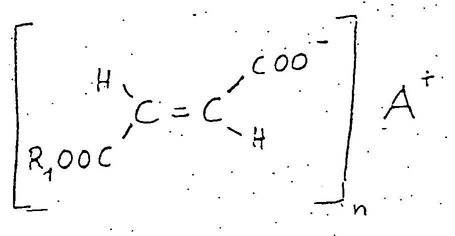
- R₁ die oben gegebene Bedeutung hat,
- A Wasserstoff, oder ein Alkali- oder Erdalkalimetallkation oder ein physiologisch verträgliches Übergangsmetallkation, vorzugsweise ausgewählt unter Li⁺, Na⁺, K⁺, Mg²⁺, Ca²⁺, Zn²⁺, Fe²⁺, Mn²⁺, ist und
n gleich 1 oder 2 ist und der Valenz von A entspricht, und worin es sich bei den mitochondrialen Erkrankungen um das Parkinson-Syndrom, die Alzheimer-Krankheit, die Chorea-Huntington-Krankheit, Retinopathia pigmentosa oder mitochondriale Enzephalomyopathie handelt, **dadurch gekennzeichnet, dass** eine Dosiseinheit der pharmazeutischen Zubereitung eine 1-500 mg Fumarsäure entsprechende Menge an Fumarsäurederivat(en) enthält.

2. Verwendung gemäß Anspruch 1, **dadurch gekennzeichnet, dass** es sich bei den Resten R₁ und R₂ um Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl; Octyl, Vinyl, Allyl, 2-Hydroxyethyl, 2- und/oder 3-Hydroxypropyl, 2-Methoxyethyl, Methoxymethyl oder 2-oder 3-Methoxypropyl handelt.

3. Verwendung gemäß einem der vorstehenden Ansprüche zur Herstellung einer pharmazeutischen Zubereitung zur oralen, parenteralen, rektalen, transdermalen oder ophthalen Verabreichung, vorzugsweise zur oralen Verabreichung.

4. Verwendung gemäß Anspruch 3, bei der die pharmazeutische Zubereitung zur oralen Verabreichung in Form von Unit-Dose-Tabletten, gegebenenfalls verkapselten oder in Sackets abgefüllten Mikrotabletten (Mikropellets) bzw. Granulat, Kapseln oder Trinklösungen vorliegt.

5. Verwendung gemäß Anspruch 4, **dadurch gekennzeichnet, dass** die festen Dosisformen mit einem magensaftresistenten Überzug versehen sind.

6. Verwendung gemäß einem der vorstehenden Ansprüche, **dadurch gekennzeichnet, dass** es sich bei dem Fumarsäurederivat um eines oder mehrere, ausgewählt aus der Gruppe, umfassend Fumarsäuredimethylester, Fumarsäurediethylester, Fumarsäuremethylethylester, Methylhydrogenfumarat, Ethylhydrogenfumarat, Calciummethylfumarat, Calciumethylfumarat, Magnesiummethylfumarat, Magnesiumethylfumarat, Zinkmethylfumarat, Zinkethylfumarat, Eisenmethylfumarat und/oder Eisenethylfumarat, handelt.

7. Verwendung gemäß Anspruch 6, **dadurch gekennzeichnet, dass** die erhaltenen Dosiseinheiten der pharmazeutischen Zubereitung einzeln oder im Gemisch bevorzugt enthalten:
| | |
|---|---|
| 10-500 mg | Dialkylfumarat, insbesondere Dimethylfumarat und/oder Diethylfumarat, |
| 10-500 mg | Calciumalkylfumarat, insbesondere Calciummethylfumarat und/oder Calciumethylfumarat, |
| 0-250 mg | Zinkalkylfumarat, insbesondere Zinkmethylfumarat und/oder Zinkethylfumarat, |
| 0-250 mg | Alkylhydrogenfumarat, insbesondere Methylhydrogenfumarat und/oder Ethylhydrogenfumarat und |
| 0-250 mg | Magnesiumalkylfumarat, insbesondere Magnesiummethylfumarat und/oder Magnesiumethylfumarat |
wobei die Summe der genannten Mengen einem Äquivalent von 500 mg, vorzugsweise 300 mg und am meisten bevorzugt 200 mg Fumarsäure entspricht.

8. Verwendung nach einem der vorstehenden Ansprüche, worin eine Dosiseinheit eine 10-300 mg Fumarsäure entsprechende Menge an Fumarsäurederivat(en) enthält.

9. Verwendung nach einem der vorstehenden Ansprüche, worin eine Dosiseinheit eine 10-200 mg Fumarsäure entsprechende Menge an Fumarsäurederivat(en) enthält.

## Claims

1. The use of individual or a mixture of fumaric acid derivatives for preparing a pharmaceutical composition for treating mitochondrial diseases wherein the fumaric acid derivatives are those selected from the group consisting of fumaric acid dialkyl esters and fumaric acid monoalkyl esters in the form of the free acid or a salt thereof, said fumaric acid dialkyl ester corresponding to the formula 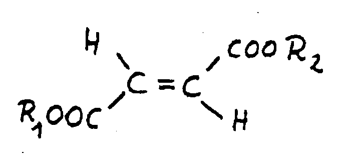 wherein R₁ and R₂ which may be the same or different independently represent a linear, branched, cyclic, saturated or unsaturated C₁₋₂₄ alkyl radical or a C₅₋₂₀ aryl radical and wherein these radicals are optionally substituted with halogen (F, Cl, Br, I), hydroxy, C₁₋₄ alkoxy, nitro or cyano, or wherein the fumaric acid monoalkyl ester corresponds to the formula 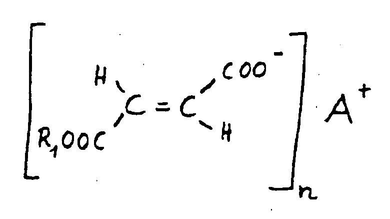 wherein
- R₁ is as defined above,
- A is hydrogen or an alkaline or alkaline earth metal cation or a physiologically acceptable transition metal cation, preferably selected from Li⁺, Na⁺, K⁺, M²⁺, C²⁺, Z²⁺, Mn²⁺ and
- n is 1 or 2 and corresponds to the valence of A, said mitochondrial diseases being Parkinson's syndrome, Alzheimer's disease, Chorea Huntington disease, retinopathia pigmentosa or mitochondrial encephalomyopathy, **characterised in that** a dosage unit of the pharmaceutical composition contains an amount of fumaric acid derivative(s) corresponding to 1 to 500 mg of fumaric acid.

2. The use according to claim 1, **characterised in that** the radicals R₁ and R₂ are methyl, ethyl, n-propyl, isopropyl, n-butyl, sec-butyl, t-butyl, pentyl, cyclopentyl, 2-ethylhexyl, hexyl, cyclohexyl, heptyl, cycloheptyl, octyl, vinyl, allyl, 2-hydroxyethyl, 2- and/or 3-hydroxypropyl, 2-methoxyethyl, methoxymethyl or 2- or 3-methoxypropyl.

3. The use according to any of the previous claims for preparing a pharmaceutical composition for oral, parenteral, rectal, transdermal or ophthal administration, preferably for oral administration.

4. The use according to claim where the pharmaceutical composition for oral administration is present in the form of unit dosage tablets, micro-tablets or micro-tablets optionally encapsulated or filled into sachets (micropellets) or granulate, capsules or solutions for drinking.

5. The use according to claim 4, **characterised in that** the solid dosage forms are provided with an enteric coating.

6. The use according to any of the previous claims, **characterised in that** the fumaric acid derivative is one or more selected from the group comprising fumaric acid dimethyl ester, fumaric acid diethyl ester, fumaric acid methyl ethyl ester, methyl hydrogen fumarate, ethyl hydrogen fumarate, calcium methyl fumarate, calcium ethyl fumarate, magnesium methyl fumarate, magnesium ethyl fumarate, zinc methyl fumarate, zinc ethyl fumarate, iron methyl fumarate and/or iron ethyl fumarate.

7. The use according to claim 6, **characterised in that** the dosage units of the pharmaceutical composition obtained preferably contain either individually or in a mixture:
| | |
|---|---|
| 10 to 500 mg | of dialkyl fumarate, especially dimethyl fumarate and/or diethyl fumarate; |
| 10 to 500 mg | of calcium alkyl fumarate, especially calcium methyl fumarate and/or calcium ethyl fumarate; |
| 0 to 250 mg | of zinc alkyl fumarate, especially zinc methyl fumarate and/or zinc ethyl fumarate; |
| 0 to 250 mg | of alkyl hydrogen fumarate, especially methyl hydrogen fumarate and/or ethyl hydrogen fumarate, and |
| 0 to 250 mg | of magnesium alkyl fumarate, especially magnesium methyl fumarate and/or magnesium ethyl fumarate |
the total of the amounts specified corresponding to an equivalent of 500 mg, preferably 300 mg and most preferably 200 mg of fumaric acid.

8. The use according to any of the previous claims, where one dosage unit contains an amount of fumaric acid derivative(s) corresponding to 10 to 300 mg of fumaric acid.

9. The use according to any of the previous claims, where one dosage unit contains an amount of fumaric acid derivative(s) corresponding to 10 to 200 mg of fumaric acid.

## Revendications

1. Utilisation de dérivés d'acide fumarique individuels ou d'un mélange de dérivés d'acide fumarique pour fabriquer une préparation pharmaceutique pour le traitement de maladies mitochondriales, dans laquelle on choisit le ou les dérivés d'acide fumarique dans le groupe constitué de dialkylesters d'acide fumarique et de monoalkylesters d'acide fumarique sous forme d'acide libre ou d'un de leurs sels, le dialkylester d'acide fumarique correspondant à la formule : 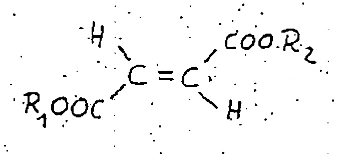 dans laquelle R₁ et R₂, qui peuvent respectivement être identiques ou différents, représentent, indépendamment l'un de l'autre, un radical alkyle en C₁₋₂₄ linéaire, ramifié, cyclique, saturé ou insaturé ou un radical alkyle en C₅₋₂₀ et ces radicaux sont éventuellement substitués par un halogène (F, Cl, Br, I), un hydroxy, un alcoxy en C₁₋₄, un nitro ou un cyano ou dans laquelle le monoalkylester d'acide fumarique correspond à la formule : 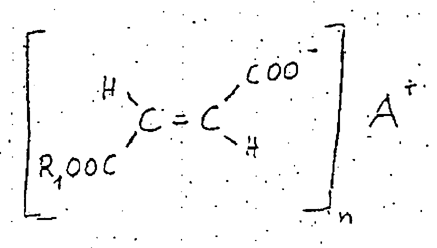
- R₁ a la signification précitée,
- A est de l'hydrogène ou un cation de métal alcalin ou alcalinoterreux ou un cation de métal de transition physiologiquement compatible, de préférence choisi parmi Li⁺, Na⁺, K⁺, Mg²⁺, Ca²⁺, Zn²⁺, Fe²⁺, Mn²⁺ et
n est égal à 1 ou 2 et correspond à la valence de A, et dans laquelle les maladies mitochondriales sont notamment le syndrome de Parkinson, la maladie d'Alzheimer, la maladie de la chorée de Huntington, de la Retinopathia pigmentosa ou l'encéphalomyopathie mitochondriale, **caractérisée en ce qu'**une unité de dosage de la préparation pharmaceutique contient une quantité de dérivé(s) d'acide fumarique correspondant à 1 à 500 mg d'acide fumarique.

2. Utilisation selon la revendication 1, **caractérisée en ce que** les radicaux R₁ et R₂ sont notamment les radicaux méthyle, éthyle, n-propyle, isopropyle, n-butyle, sec-butyle, t-butyle, pentyle, cyclopentyle, 2-éthylhexyle, hexyle, cyclohexyle, heptyle, cycloheptyle, octyle, vinyle, allyle, 2-hydroxyéthyle, 2- et/ou 3-hydroxypropyle, 2-méthoxyéthyle, méthoxyméthyle ou 2-ou 3-méthoxypropyle.

3. Utilisation selon l'une quelconque des revendications précédentes pour fabriquer une préparation pharmaceutique à administrer par voie orale, parentérale, rectale, transcutanée ou ophtalmique, de préférence par voie orale.

4. Utilisation selon la revendication 3, dans laquelle la préparation pharmaceutique pour administration, orale sous la forme de comprimés en doses unitaires, de microcomprimés (micropellets) éventuellement encapsulés ou ensachés ou de granulés, de capsules ou de solutions buvables.

5. Utilisation selon la revendication 4, **caractérisée en ce que** les formes de dosage solides sont pourvues d'un enrobage résistant aux sucs gastriques.

6. Utilisation selon l'une quelconque des revendications précédentes, **caractérisée en ce que** le dérivé d'acide fumarique représente une ou plusieurs substances choisies dans le groupe comprenant le diméthylester d'acide fumarique, le diéthylester d'acide fumarique, le méthyléthylester d'acide fumarique, l'hydrogénofumarate de méthyle, l'hydrogénofumarate d'éthyle, le méthylfumarate de calcium, l'éthylfumarate de calcium, le méthylfumarate de magnésium, l'éthylfumarate de magnésium, le méthylfumarate de zinc, l'éthylfumarate de zinc, le méthylfumarate de fer et/ou l'éthylfumarate de fer.

7. Utilisation selon la revendication 6, **caractérisée en ce que** les unités de dosage obtenues de la préparation pharmaceutique contiennent de préférence individuellement ou en mélange :
10 à 500 mg de fumarate de dialkyle, en particulier de fumarate de diméthyle et/ou de fumarate de diéthyle,
10 à 500 mg d'alkylfumarate de calcium, en particulier le méthylfumarate de calcium et/ou l'éthylfumarate de calcium,
0 à 250 mg d'alkylfumarate de zinc, en particulier le méthylfumarate de zinc et/ou l'éthylfumarate de zinc,
0 à 250 mg d'drogériofumarate d'alkyle, en particulier l'hydrogénofumarate de méthyle et/ou l'hydrogénofumarate d'éthyle, et
0 à 250 mg d'alkylfumarate de magnésium, en particulier le méthylfumarate de magnésium et/ou l'éthylfumarate de magnésium,
la somme des quantités citées correspondant à un équivalent de 500 mg, de préférence de 300 mg et, mieux encore, de 200 mg d'acide fumarique.

8. Utilisation selon l'une quelconque des revendications précédentes, dans laquelle une unité de dosage contient une quantité de dérivé(s) d'acide fumarique correspondant à 10 à 300 mg d'acide fumarique.

9. Utilisation selon l'une quelconque des revendications précédentes, dans laquelle une unité de dosage contient une quantité de dérivé(s) d'acide fumarique correspondant à 10 à 200 mg d'acide fumarique.
